# EUROPÄISCHE PATENTSCHRIFT

(11) **EP 2 637 549 B1**
(45) Veröffentlichungstag und Bekanntmachung des Hinweises auf die Patenterteilung: **12.06.2019**
(21) Anmeldenummer: 11805398.2
(22) Anmeldetag: 11.11.2011
(51) Int. Cl.: A61B 1/00, A61B 1/06, A61B 1/07, G02B 23/24

(54) **ENDOSKOP MIT SCHWENKBARER BLICKRICHTUNG**
ENDOSCOPE WITH PIVOTABLE VIEWING DIRECTION
ENDOSCOPE À UNE DIRECTION DE VISÉE BASCULANTE

(30) Priorität: 11.11.2010 DE 102010050933
(43) Veröffentlichungstag der Anmeldung: 18.09.2013
(73) Patentinhaber: Karl Storz SE & Co. KG, 78532 Tuttlingen (DE)
(72) Erfinder: EISENKOLB, Peter, 78532 Tuttlingen (DE); HESELER, Stefan, 78647 Trossingen (DE)
(86) Internationale Anmeldenummer: PCT/EP2011/005683
(87) Internationale Veröffentlichungsnummer: WO 2012/062476

(56) Entgegenhaltungen:
- EP-A1- 2 415 388
- EP-A1- 2 415 391
- WO-A1-01/39657
- DE-U1- 20 013 186
- US-A- 3 896 793
- US-A- 4 403 273

## Beschreibung

Die vorliegende Erfindung bezieht sich auf ein Endoskop mit schwenkbare Blickrichtiung.

Neben Endoskopen für medizinische und nicht-medizinische technische Anwendungen, deren Blickrichtung parallel zur Längsachse des Schafts des Endoskops ist, wurden bereits früh Endoskope mit anderen feststehenden Blickrichtungen entwickelt. Mit der Blickrichtung eines Endoskops ist hier und im Folgenden immer die Richtung vom distalen Ende des Endoskops aus gemeint, in der ein Gegenstand liegt, der in der Mitte des mittels des Endoskops erfassten Bilds erscheint. Bei vielen Anwendungen ist jedoch eine feste Blickrichtung nachteilig. Im ungünstigsten Fall muss beispielsweise während eines medizinischen Eingriffs mehrfach das Endoskop gewechselt werden. In solchen Fällen ist die Verwendung eines Endoskops mit einer in situ einstellbaren bzw. verstellbaren Blickrichtung vorteilhaft.

Die Beobachtung eines Gegenstands in einem Hohlraum mittels eines Endoskops setzt in der Regel eine Beleuchtung des Gegenstands voraus. Dazu weist ein Endoskop beispielsweise Lichtwellenleiter, insbesondere Glasfasern, auf, mittels derer Beleuchtungslicht vom proximalen Ende des Endoskops entlang des Schafts zum distalen Ende des Endoskops übertragen wird. Lichtaustrittsflächen der Lichtwellenleiter am distalen Ende des Endoskops sind so angeordnet und ausgebildet, dass das gesamte Sichtfeld bzw. Blickfeld ausreichend ausgeleuchtet wird.

Um das gesamte Sichtfeld für alle möglichen Blickrichtungen auszuleuchten, sind beispielsweise mehrere Lichtaustrittsflächen vorgesehen, durch die Beleuchtungslicht in unterschiedliche Richtungen austritt. Lichtwellenleiter zu diesen Lichtaustrittsflächen weisen teilweise unmittelbar proximal der Lichtaustrittsfläche Krümmungen mit kleinen Radien auf. Spätestens beim Übergang zu immer kleineren Schaftdurchmessern werden die Krümmungsradien jedoch bald so gering, dass ein Bruch von Lichtwellenleitern nicht mehr die Ausnahme, sondern die Regel wäre. Außerdem gehen bei zu kleinem Krümmungsradius die lichtleitenden Eigenschaften eines Lichtwellenleiters verloren.

In der DE 600 15 375 T2 ist eine Anordnung mehrerer Prismen beschrieben. Eines der Prismen ist um eine Achse rotierbar, um Beleuchtungslicht in eine einstellbare Blickrichtung zu werfen. Diese Anordnung ist jedoch allenfalls mit hohem Aufwand weiter miniaturisierbar. Bei Endoskopen mit immer dünneren Schäften sind alternative, weiter miniaturisierbare Konzepte erforderlich.

Dokument US 3,896,793 offenbart ein Endoskop mit einstellbarer Blickrichtung mit einem Beleuchtungslichtfaserbündel.

Eine Aufgabe der vorliegenden Erfindung besteht darin, ein verbessertes Endoskop mit schwenkbarer Blickrichtung zu schaffen.

Diese Aufgabe wird durch den Gegenstand des unabhängigen Anspruchs 1 gelöst.

Weiterbildungen sind in den abhängigen Ansprüchen angegeben.

Die vorliegende Erfindung beruht auf der Idee, bei einem Endoskop mit einstellbarer Blickrichtung eine Mehrzahl von zumindest teilweise nebeneinander im Beleuchtungsstrahlengang angeordneten reflektierenden Flächen vorzusehen, um alle möglichen Sichtfelder des Endoskops auszuleuchten. Durch die Mehrzahl reflektierender Flächen kann das Beleuchtungslicht aufgefächert werden, um zumindest in einer Richtung einen großen Winkelbereich zu beleuchten.

Ein Endoskop mit relativ zum Endoskop schwenkbarer Blickrichtung umfasst einen Beleuchtungsstrahlengang zum Übertragen von Beleuchtungslicht und eine Mehrzahl von reflektierenden Flächen zum Reflektieren von Beleuchtungslicht, wobei die reflektierenden Flächen bezogen auf die vorgesehene Lichtausbreitungsrichtung von Beleuchtungslicht im Beleuchtungsstrahlengang zumindest teilweise nebeneinander im Beleuchtungsstrahlengang angeordnet sind.

Die Blickrichtung des Endoskops ist insbesondere um eine Schwenkachse schwenkbar, die senkrecht zur Längsachse des Endoskops oder zumindest nicht parallel zur Längsachse des Endoskops ist. Das Endoskop kann eine oder mehrere Leuchtdioden oder eine oder mehrere andere Lichtquellen am distalen Ende oder am proximalen Ende aufweisen. In diesem Fall erstreckt sich der Beleuchtungsstrahlengang von der bzw. den Lichtquellen bis zu einem oder mehreren Beleuchtungsfenstern am distalen Ende des Endoskops. Alternativ erstreckt sich der Beleuchtungsstrahlengang von einer Kupplung am proximalen Ende des Endoskops bis zu einem oder mehreren Beleuchtungsfenstern am distalen Ende des Endoskops, wobei die Kupplung ausgebildet ist, um das Endoskop mittels eines Lichtleitkabels mit einer Lichtquelle zu koppeln. Zur Übertragung von Beleuchtungslicht vom proximalen Ende zum distalen Ende des Endoskops weist dieses beispielsweise einen Lichtwellenleiter, ein Bündel von Lichtwellenleitern oder einen anderen Lichtleiter auf.

Der Querschnitt des Beleuchtungsstrahlengangs ist insbesondere unmittelbar lichtstromaufwärts der reflektierenden Flächen einfach zusammenhängend. Das Endoskop kann mehrere Beleuchtungsstrahlengänge aufweisen, wobei innerhalb jedes einzelnen Beleuchtungsstrahlengangs eine Mehrzahl reflektierender Flächen bezogen auf die vorgesehene Lichtausbreitungsrichtung zumindest teilweise nebeneinander angeordnet ist. Insbesondere weist das Endoskop zwei oder mehr Beleuchtungsstrahlengänge an gegenüberliegenden Seiten eines Beobachtungsstrahlengangs auf, wobei am distalen Ende des Endoskops Lichtaustrittsflächen eines ersten Beleuchtungsstrahlengangs und Lichtaustrittsflächen eines zweiten Beleuchtungsstrahlengangs an gegenüberliegenden Seiten einer Lichteintrittsfläche des Beobachtungsstrahlengangs angeordnet sind.

Die vorgesehene Lichtausbreitungsrichtung von Beleuchtungslicht ist von der Lichtquelle oder vom proximalen Ende des Endoskops zu der oder den Lichtaustrittsflächen am distalen Ende des Endoskops. Die reflektierenden Flächen erstrecken sich insbesondere zumindest teilweise im Wesentlichen parallel zur vorgesehenen Lichtausbreitungsrichtung. Da Beleuchtungslicht real von einer ausgedehnten, nicht punktförmigen Lichtquelle ausgeht, breitet es sich in einem Beleuchtungsstrahlengang mit endlichem Querschnitt nie vollkommen parallel aus. Als Lichtausbreitungsrichtung wird deshalb beispielsweise die mittlere Lichtausbreitungsrichtung innerhalb des Querschnitts des Beleuchtungsstrahlengangs angesehen.

Insbesondere unterteilen die reflektierenden Flächen den Querschnitt des Beleuchtungsstrahlengangs in mehrere Teilquerschnitte. Durch eine auffächernde Wirkung der reflektierenden Flächen auf das Beleuchtungslicht kann die Lichtausbreitungsrichtung (wiederum im Sinne einer mittleren Lichtausbreitungsrichtung innerhalb des betreffenden Teilquerschnitts) zwischen jedem Paar benachbarter reflektierender Flächen anders sein.

Das Kriterium der Anordnung einer ersten reflektierenden Fläche und einer zweiten reflektierenden Fläche zumindest teilweise nebeneinander ist insbesondere auf die Lichtausbreitungsrichtungen unmittelbar lichtstromaufwärts und unmittelbar lichtstromabwärts der lichtstromaufwärts angeordneten Ränder der reflektierenden Flächen bezogen. Dabei ist die Lichtausbreitungsrichtung unmittelbar lichtstromabwärts des lichtstromaufwärtigen Rands einer reflektierenden Fläche nur unwesentlich verändert, weil nur ein geringer Teil des Beleuchtungslichts bereits an der reflektierenden Fläche reflektiert ist.

Eine erste reflektierende Fläche und eine zweite reflektierende Fläche sind bezogen auf die vorgesehene Lichtausbreitungsrichtung von Beleuchtungslicht im Beleuchtungsstrahlengang zumindest teilweise nebeneinander im Beleuchtungsstrahlengang angeordnet, wenn sie in Bezug auf die Lichtausbreitungsrichtung unmittelbar lichtstromaufwärts und bezogen auf die Lichtausbreitungsrichtung unmittelbar lichtstromabwärts des lichtstromaufwärtigen Rands der ersten reflektierenden Fläche und bezogen auf die Lichtausbreitungsrichtung unmittelbar lichtstromaufwärts und bezogen auf die Lichtausbreitungsrichtung unmittelbar lichtstromabwärts des lichtstromaufwärtigen Rands der zweiten reflektierenden Fläche nebeneinander angeordnet sind. Bezogen auf eine Richtung liegen zwei reflektierende Flächen nebeneinander, wenn eine zu der Richtung senkrechte Ebene beide reflektierende Flächen schneidet. Insbesondere werden zu den Lichtausbreitungsrichtungen senkrechte Ebenen betrachtet, die die reflektierenden Flächen an deren lichtstromaufwärtigen Rändern oder nahe deren lichtstromaufwärtigen Rändern schneiden.

Bereits einem intuitiven Verständnis entsprechend sind die reflektierenden Flächen der in der oben erwähnten DE 600 15 375 T2 beschriebenen Prismen nicht zumindest teilweise nebeneinander im Sinne der vorliegenden Erfindung angeordnet. Beleuchtungslicht kann nicht gleichzeitig auf beide reflektierenden Flächen der Prismen fallen. Vielmehr wird Beleuchtungslicht zunächst von der reflektierenden Fläche eines ersten Prismas um 90 Grad abgelenkt und dann von der reflektierenden Fläche eines zweiten Prismas wiederum um 90 Grad abgelenkt. Die Anordnung ist dazu ausgebildet, dass Beleuchtungslicht, das auf die zweite reflektierende Fläche trifft, zuvor von der ersten reflektierenden Fläche um 90 Grad abgelenkt wurde. Die reflektierenden Flächen sind also bezogen auf die Lichtausbreitungsrichtung nicht nebeneinander, sondern hintereinander angeordnet.

Auch bezogen auf die oben angegebenen Kriterien für eine Anordnung reflektierender Flächen zumindest teilweise nebeneinander sind die reflektierenden Flächen der Prismen gemäß DE 600 15 375 T2 nicht nebeneinander angeordnet. Es gibt zwar Ebenen, die von den reflektierenden Flächen beider Prismen geschnitten werden, jedoch gibt es beispielsweise keine Ebene senkrecht zur Lichtausbreitungsrichtung zwischen den beiden reflektierenden Flächen, die beide reflektierenden Flächen schneidet.

Die Mehrzahl reflektierender Flächen ermöglicht zumindest innerhalb einer Richtung eine Aufweitung bzw. Auffächerung des Beleuchtungslichts und damit eine Ausleuchtung aller möglicher Sichtfelder eines Endoskops mit schwenkbarer Blickrichtung. Die Anordnung einer Mehrzahl reflektierender Flächen kann mit geringem oder verhältnismäßigem Aufwand miniaturisierbar sein. Da die reflektierenden Flächen starr bzw. unbewegt angeordnet sein können, kann auch eine große mechanische Robustheit und eine Unempfindlichkeit gegenüber Sterilisationsprozessen im Autoklaven realisierbar sein.

Bei einem Endoskop, wie es hier beschrieben ist, können die reflektierenden Flächen fächerförmig angeordnet sein.

Die reflektierenden Flächen sind insbesondere fächerförmig angeordnet, wenn ihre Abstände in eine Richtung zunehmen. Insbesondere nehmen ihre Abstände in der Lichtausbreitungsrichtung zu.

Soweit die reflektierenden Flächen eben sind, sind sie insbesondere dann fächerförmig angeordnet, wenn alle Ebenen, in denen die reflektierenden Flächen liegen sich in einer einzigen Gerade oder in mehreren parallelen Geraden schneiden, wobei der maximale Abstand zweier paralleler Schnittgeraden nicht größer ist als die linearen Abmessungen der reflektierenden Flächen in einer Richtung senkrecht zu den Schnittgeraden oder nicht größer als die Hälfte, ein Drittel oder ein Fünftel dieser Abmessung.

Die fächerförmige Anordnung der reflektierenden Flächen ermöglicht eine Aufweitung bzw. Auffächerung des Beleuchtungslichts in einer Richtung. Durch die Anzahl, Größe und Gestalt der reflektierenden Flächen und ihre Winkelabstände ist die Verteilung des Beleuchtungslichts einstellbar.

Bei einem Endoskop, wie es hier beschrieben ist, können die Flächennormalen ebener reflektierender Flächen der Mehrzahl von reflektierenden Flächen in einer Ebene senkrecht zu einer Schwenkachse der Blickrichtung liegen.

Insbesondere sind alle reflektierenden Flächen der Mehrzahl reflektierender Flächen eben und die Flächennormalen aller reflektierender Flächen liegen in einer Ebene senkrecht zur Schwenkachse der Blickrichtung. Alternativ ist nur eine Teilmenge der reflektierenden Flächen eben, wobei die Flächennormalen aller ebener reflektierender Flächen oder zumindest die Flächennormalen mehrerer ebener reflektierender Flächen in einer Ebene senkrecht zu einer Schwenkachse der Blickrichtung liegen.

Bei einem Endoskop, wie es hier beschrieben ist, können lokale Flächennormalen reflektierender Flächen in mittleren Bereichen der reflektierenden Flächen in einer Ebene senkrecht zu einer Schwenkachse der Blickrichtung liegen.

Die Ausbildung und Anordnung ebener oder gekrümmter reflektierender Flächen derart, dass die Flächennormalen bzw. im Fall gekrümmter reflektierender Flächen die lokalen Flächennormalen in mittleren Bereichen der reflektierenden Flächen in einer Ebene senkrecht zur Schwenkachse der Blickrichtung liegen, kann eine präzise Auffächerung des Beleuchtungslichts in der Schwenkrichtung ermöglichen. Durch die Anzahl, Größe, Gestalt und insbesondere auch Krümmung der reflektierenden Flächen kann die Verteilung des Beleuchtungslichts eingestellt werden.

Bei einem Endoskop, wie es hier beschrieben ist, kann eine reflektierende Fläche der Mehrzahl von reflektierenden Flächen einen Reflexionsgrad von höchstens 0,75 aufweisen.

Insbesondere weist die reflektierende Fläche oder weisen mehrere oder alle reflektierenden Flächen der Mehrzahl von reflektierenden Flächen einen Reflexionsgrad von höchstens 0,75 oder höchstens 0,50 oder höchstens 0,25 auf. Ein Reflexionsgrad der reflektierenden Flächen, der deutlich kleiner als 1 ist, kann eine weiter verfeinerte Auffächerung des Beleuchtungslichts und eine besonders hohe Gleichmäßigkeit der Intensität des Beleuchtungslichts ermöglichen. Dazu kann insbesondere beitragen, dass Beleuchtungslicht nicht nur zwischen zwei nächst benachbarten reflektierenden Flächen hin- und herreflektiert wird, sondern teilweise nacheinander an mehreren verschiedenen reflektierenden Flächen reflektiert wird.

Ein Endoskop, wie es hier beschrieben ist, kann eine Lichtverteilungseinrichtung aufweisen, die eine Mehrzahl transparenter Körper umfasst, wobei die reflektierenden Flächen an Grenzflächen zwischen jeweils zwei aneinanderangrenzenden transparenten Körpern vorliegen.

Die transparenten Körper können Prismen aus einem transparenten Material sein.

Mit einem Prisma ist insbesondere ein Prisma im strengen Sinne der Geometrie gemeint, also ein Körper mit zwei gegenüberliegenden, parallelen und gleichen Begrenzungsflächen, dessen übrige Begrenzungsflächen Parallelogramme sind. In der Optik wird der Begriff des Prismas oft im weiteren Sinne verwendet, um transparente Körper zu bezeichnen, deren Oberfläche zumindest zwei nicht-parallele ebene Abschnitte aufweisen, durch die Licht in einer ersten Richtung eintritt und in einer zweiten Richtung austritt. In weiterer Verallgemeinerung kann die Lichtverteilungseinrichtung des hier beschriebenen Endoskops eine Mehrzahl nicht-prismatischer transparenter Körper aufweisen.

Die Ausbildung der reflektierenden Flächen an Grenzflächen zwischen miteinander beispielsweise verkitteten transparenten Körpern kann besonders robust und gleichzeitig weitgehend miniaturisierbar sein. Die reflektierenden Flächen sind durch die transparenten Körper geschützt, die Lichtverteilungseinrichtung bildet ein kompaktes und mechanisch robustes Bauteil, das leicht montierbar ist.

Bei einem Endoskop mit einer Lichtverteilungseinrichtung, wie es hier beschrieben ist, können an einzelne transparente Körper der Lichtverteilungseinrichtung angrenzende Abschnitte einer Lichtaustrittsfläche der Lichtverteilungseinrichtung eben sein.

Alternativ können bei einem Endoskop mit einer Lichtverteilungseinrichtung, wie es hier beschrieben ist, an einzelne transparente Körper der Lichtverteilungseinrichtung angrenzende Abschnitte einer Lichtaustrittsfläche der Lichtverteilungseinrichtung gekrümmt sein.

In einem einfachen Fall weist jeder transparente Körper eine ebene oder beispielsweise zylindrisch oder elliptisch oder sphärisch oder torisch gekrümmte Fläche auf, die an gegenüberliegenden Rändern an zwei verschiedene reflektierende Flächen der Mehrzahl von reflektierenden Flächen angrenzt, und die einen Abschnitt der Lichtaustrittsfläche der Lichtverteilungseinrichtung bildet. Durch die ebene oder gekrümmte Ausgestaltung dieser Abschnitte der Lichtaustrittsfläche kann die Verteilung des Beleuchtungslichts bzw. seiner Intensität weiter verbessert werden.

Bei einem Endoskop mit einer Lichtverteilungseinrichtung, wie es hier beschrieben ist, können an einzelne transparente Körper der Lichtverteilungseinrichtung angrenzende Abschnitte einer Lichteintrittsfläche der Lichtverteilungseinrichtung eben sein.

Alternativ sind an einzelne transparente Körper der Lichtverteilungseinrichtung angrenzende Abschnitte einer Lichteintrittsfläche der Lichtverteilungseinrichtung gekrümmt.

In einem einfachen Fall weist ein transparenter Körper einen ebenen, zylindrisch, torisch, elliptisch, sphärisch oder anders gekrümmten Oberflächenabschnitt auf, der an gegenüberliegenden Rändern an zwei reflektierende Flächen angrenzt und einen Teil der Lichteintrittsfläche der Lichtverteilungseinrichtung bildet. Durch die Ausgestaltung der Lichteintrittsfläche kann die Verteilung des Beleuchtungslichts verbessert werden. Insbesondere kann eine konvex gekrümmte Lichteintrittsfläche der Lichtverteilungseinrichtung kollimierend wirken. Alternativ kann durch eine entsprechend gekrümmte oder abschnittsweise oder insgesamt eben ausgebildete Lichteintrittsfläche der Lichtverteilungseinrichtung eine Divergenz oder Konvergenz des Beleuchtungslichts eingestellt werden. Die Lichteintrittsfläche kann ferner mit einer Leuchtdiode oder einer anderen Lichtquelle oder einer Lichtaustrittsfläche eines Lichtleiters verschweißt, verklebt oder auf andere Weise gefügt sein. Dies ermöglicht eine dauerhafte und dauerhaft präzise Ausrichtung der Lichtquelle bzw. der Lichtaustrittsfläche des Lichtleiters relativ zur Lichtverteilungseinrichtung.

Bei einem Endoskop, wie es hier beschrieben ist, können die reflektierenden Flächen zumindest entweder um eine Achse schwenkbar oder entlang eines Wegs verschiebbar sein.

Selbst eine geringfügige Verschwenkung oder Verschiebung kann bei entsprechender Ausgestaltung der reflektierenden Flächen eine Veränderung des mittels der reflektierenden Flächen ausgeleuchteten Raumwinkelbereichs ermöglichen. Insbesondere wenn das Endoskop eine Lichtverteilungseinrichtung aufweist, wie sie oben beschrieben ist, kann diese gleichzeitig robust und auf einfache und präzise Weise schwenkbar oder verschiebbar ausgebildet sein.

Bei einem Endoskop, bei dem die reflektierenden Flächen zumindest entweder um eine Achse schwenkbar oder entlang eines Wegs verschiebbar sind, ist insbesondere die Beleuchtung der reflektierenden Flächen von der durch Schwenken oder Schieben der reflektierenden Flächen eingestellten Position der reflektierenden Flächen abhängig.

Insbesondere kann eingestellt werden, auf welche reflektierende Fläche Beleuchtungslicht fällt oder in welchem Winkel Beleuchtungslicht auf eine oder in welchen Winkeln Beleuchtungslicht auf mehrere reflektierende Flächen fällt. Dadurch kann der mit Beleuchtungslicht ausgeleuchtete Raumwinkelbereich eingestellt werden.

### Kurzbeschreibung der Figuren

Nachfolgend werden Ausführungsformen anhand der beigefügten Figuren näher erläutert. Es zeigen:
- Figur 1: eine schematische Darstellung eines Endoskops mit einstellbarer Blickrichtung;
- Figur 2: eine schematische Darstellung einer Ausführungsform eines distalen Endes eines Endoskops;
- Figur 3: eine schematische Darstellung einer weiteren Ausführungsform eines distalen Endes eines Endoskops;
- Figur 4: eine schematische Darstellung einer weiteren Ausführungsform eines distalen Endes eines Endoskops;
- Figur 5: eine schematische Darstellung einer weiteren Ausführungsform eines distalen Endes eines Endoskops;
- Figur 6: eine schematische Darstellung einer weiteren Ausführungsform eines distalen Endes eines Endoskops;
- Figur 7: eine schematische Darstellung einer weiteren Ausführungsform eines distalen Endes eines Endoskops.

### Beschreibung von Ausführungsformen

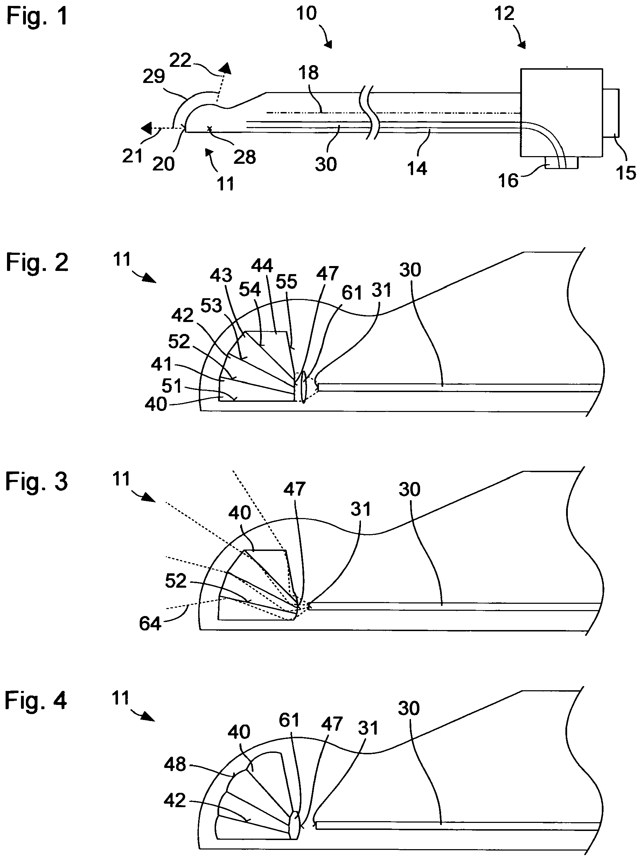

Figur 1 zeigt eine schematische Darstellung eines Endoskops 10 mit einem distalen Ende 11, einem proximalen Ende 12 und einem starren Schaft 14, der sich vom distalen Ende 11 bis zum proximalen Ende 12 erstreckt. Alternativ ist der Schaft 14 flexibel oder teilweise flexibel. Der Querschnitt des Schafts 14 oder zumindest die äußere Kontur des Querschnitts des Schafts 14 ist zwischen dem distalen Ende 11 und dem proximalen Ende 12 konstant oder im Wesentlichen konstant. Insbesondere ist die Kontur des Querschnitts des Schafts 14 kreisförmig oder elliptisch. In diesem Fall ist die in Figur 1 dargestellt Längsachse 18 des Endoskops 10 die Symmetrieachse der Mantelfläche des Schafts 14 zwischen dem distalen Ende 12 und dem proximalen Ende 11. Bei einer zylindrischen Mantelfläche des Schafts 14 ist die Längsachse 18 auch die Menge der Mittelpunkte oder Flächenschwerpunkte der Querschnitte des Schafts 14 zwischen dem distalen Ende 12 und dem proximalen Ende 11. Bei einer kreiszylindrischen Mantelfläche des Schafts 14 ist die Längsachse 18 auch die Symmetrieachse der Mantelfläche.

Am distalen Ende 12 weicht die Gestalt des Schafts 14 von der Zylindersymmetrie ab, wie dies beispielhaft in Figur 1 dargestellt ist. Insbesondere weist der Schaft 14 am distalen Ende 12 eine Öffnung auf, die durch ein transparentes Fensterbauteil mit einer gewölbten Oberfläche 20 verschlossen ist. Insbesondere verschließt das Fensterbauteil 20 die Öffnung hermetisch dicht. Die Oberfläche 20 des Fensterbauteils hat beispielsweise die Gestalt eines Ausschnitts eines Kreiszylindermantels, wobei die Symmetrieachse des Kreiszylinders senkrecht zur Längsachse 18 des Endoskops 10 und zur Zeichenebene der Figur 1 ist. Alternativ hat die Oberfläche 20 des transparenten Fensterbauteils die Gestalt eines Ausschnitts einer Kugeloberfläche oder eines rotationssymmetrischen oder nicht rotationssymmetrischen Ellipsoids.

Am distalen Enden 12 des Endoskops 10 sind im Schaft 14 optische Einrichtungen angeordnet, die in Figur 1 nicht dargestellt sind. Diese optischen Einrichtungen ermöglichen eine Variation der Blickrichtung des Endoskops zwischen einer ersten extremen Blickrichtung 21 und einer zweiten extremen Blickrichtung 22. Die Blickrichtung ist zwischen den beiden extremen Blickrichtungen 21, 22 um eine Schwenkachse 28 schwenkbar, die senkrecht zur Zeichenebene der Figur 1 ist. Die Blickrichtung ist jeweils die Richtung bezogen auf das distale Ende 12 des Endoskops 10, in der ein Gegenstand liegt, der in der Mitte eines mittels des Endoskops 10 erfassten Bilds erscheint.

Bei dem in Figur 1 dargestellten Beispiel ist die erste extreme Blickrichtung 21 parallel oder im Wesentlichen parallel zur Längsachse 18 des Endoskops 10. Zwischen den extremen Blickrichtungen 21, 22 liegt ein Winkelbereich 29, der bei dem dargestellten Beispiel ca. 120 Grad umfasst. Innerhalb dieses Winkelbereichs ist die Blickrichtung des Endoskops 10 insbesondere kontinuierlich verstellbar.

Am proximalen Ende 11 weist das Endoskop 10 eine erste Kupplung 15 zum optischen Koppeln des Endoskops 10 mit einer Kamera oder ein Okular sowie eine zweite Kupplung 16 zum Koppeln des Endoskops 10 mit einer Lichtquelle über ein Lichtleitkabel auf. Von der zweiten Kupplung 16 führen ein oder mehrere Lichtleiter 30 durch den Schaft 14 bis zum distalen Ende 11 des Endoskops 10. Von einer Lichtquelle erzeugtes Beleuchtungslicht kann über ein Lichtleitkabel, die zweite Kupplung 16 und den oder die Lichtleiter 30 zum distalen Ende 11 des Endoskops 10 übertragen werden.

Die Figuren 2 bis 7 zeigen schematische Darstellungen mehrerer alternativer Ausführungsformen des distalen Endes 11 des oben anhand der Figur 1 dargestellten Endoskops 10. Die Figuren 2 bis 7 zeigen jeweils in stark vereinfachter Darstellung einen Schnitt entlang einer Ebene parallel zur Zeichenebene der Figur 1. Der Schaft bzw. das Gehäuse des Endoskops 10 ist lediglich durch einen Umriss angedeutet. Wellen, Gelenke und andere mechanische Einrichtungen zum Halten, Führen und/oder Bewegen der dargestellten optischen Einrichtungen sind zugunsten der Übersichtlichkeit nicht dargestellt.

Wie bereits erwähnt, umfasst jede der nachfolgend anhand der Figuren 2 bis 7 dargestellten Ausführungsformen einen Lichtleiter 30, der sich von der zweiten Kupplung 16 am proximalen Ende 12 bis zum distalen Ende 11 des Endoskops 10 erstreckt. Der Lichtleiter 30 umfasst beispielsweise einen Lichtwellenleiter oder ein Bündel von Lichtwellenleitern. Der Lichtleiter 30 ist zum Übertragen von Beleuchtungslicht zum distalen Ende 11 des Endoskops 10 vorgesehen und ausgebildet. Am distalen Ende 11 des Endoskops 10 weist der Lichtleiter 30 eine Lichtaustrittsfläche 31 zum Auskoppeln von Beleuchtungslichts auf. Am distalen Ende 11 des Endoskops 10 wird das Beleuchtungslicht mittels der nachfolgend anhand der Figuren 2 bis 7 dargestellten Einrichtungen in einen vorbestimmten Raumwinkelbereich verteilt.

Figur 2 zeigt eine schematische Darstellung einer Ausführungsform mit einer Lichtverteilungseinrichtung 40 am distalen Ende 11 des Endoskops 10. Die Lichtverteilungseinrichtung 40 umfasst eine Mehrzahl von Prismen 41, 42, 43, 44 aus einem oder mehreren verschiedenen transparenten Materialien. Die Prismen 41, 42, 43, 44 sind beispielsweise mittels eines Kitts, eines Klebstoffs oder auf andere Weise zu der Lichtverteilungseinrichtung 40 zusammengefügt. Zwischen jeweils zwei benachbarten Prismen 41, 42, 43, 44 ist jeweils eine reflektierende Fläche 52, 53, 54 angeordnet. An den äußersten Prismen 41, 44 sind den reflektierenden Flächen 52, 54 gegenüber weitere reflektierende Flächen 51, 55 angeordnet. Die Lichtverteilungseinrichtung 40 weist eine Lichteintrittsfläche 47 auf, die über einen Kollimator mit der Lichtaustrittsfläche 31 des Lichtleiters 30 gekoppelt ist. Die Lichteintrittsfläche 47 umfasst mehrere Teilbereiche, die jeweils durch eines der Prismen 41, 42, 43, 44 gebildet werden.

Der Kollimator 61 ist ausgebildet und angeordnet, um aus der Lichtaustrittsfläche 31 des Lichtleiters 30 austretendes Beleuchtungslicht zu kollimieren. Dazu ist die Lichtaustrittsfläche 31 des Lichtleiters 30 insbesondere am Fokus des Kollimators 61 angeordnet.

Die reflektierenden Flächen 51, 52, 53, 54, 55 sind insbesondere eben und weisen ihren kleinsten gegenseitigen Abstand nahe der Lichteintrittsfläche 47 auf. Jede einzelne reflektierende Fläche 51, 52, 53, 54, 55 kann teilweise transparent sein bzw. einen Reflexionsgrad aufweisen, der beispielsweise im Bereich von 0,2 bis 0,4 oder im Bereich von 0,4 bis 0,6 oder im Bereich von 0,6 bis 0,8 liegt. Insbesondere weisen die äußeren reflektierenden Flächen 51, 55 einen hohen Reflexionsgrad von 0,9 oder mehr und die reflektierenden Flächen 52, 53, 54 zwischen den Prismen 41, 42, 43, 44 jeweils einen mittleren Reflexionsgrad im Bereich von 0,3 bis 0,7 auf.

Alternativ weisen beispielsweise alle reflektierenden Flächen 51, 52, 53, 54, 55 einen hohen Reflexionsgrad von 0,9 oder mehr auf. Durch Nutzung von Totalreflexion ist ein Reflexionsgrad von 1 erreichbar. Dazu können abweichend von der Darstellung in Figur 2 Spalte zwischen den Prismen 41, 42, 43, 44 vorgesehen sein, in denen ein Gas oder ein anderes Medium mit einem Brechungsindex, der kleiner ist als die Brechungsindizes der Materialien der Prismen 41, 42, 43, 44, angeordnet ist.

Von der Lichtaustrittsfläche 31 des Lichtleiters 30 ausgehendes Beleuchtungslicht wird vom Kollimator 61 kollimiert und tritt an der Lichteintrittsfläche 47 in die Lichtverteilungseinrichtung ein. Durch Reflexionen an den fächerförmig angeordneten reflektierenden Flächen 51, 52, 53, 54, 55 wird das Beleuchtungslicht in einer Richtung parallel zur Zeichenebene der Figur 2 aufgefächert, um einen Raumbereich bzw. einen Raumwinkelbereich auszuleuchten, der in Richtung parallel zur Zeichenebene der Figur 2 einen wesentlich größeren Winkel einnimmt als in der Richtung senkrecht zur Zeichenebene der Figur 2. Damit ermöglicht die Lichtverteilungseinrichtung 40 eine Verteilung des Beleuchtungslichts in alle möglichen, den Blickrichtungen 21 bis 24 entsprechende Sichtfelder des Endoskops 10.

Figur 3 zeigt eine schematische Darstellung einer weiteren Ausführungsform, die in einigen Merkmalen der oben anhand der Figur 2 dargestellten Ausführungsform ähnelt. Insbesondere ist eine Lichtverteilungseinrichtung 40 vorgesehen, die mehrere Prismen und mehrere reflektierende Flächen umfasst. Im Sinne einer übersichtlichen Darstellung sind in Figur 3 einige Merkmale, die entsprechenden Merkmalen der Ausführungsform der Figur 2 ähneln, nicht mit Bezugszeichen versehen.

Im Gegensatz zu der Ausführungsform der Figur 2 ist kein Kollimator vorgesehen. Stattdessen ist die Lichteintrittsfläche 47 konvex gekrümmt und weist eine kollimierende oder zumindest die Divergenz des Beleuchtungslichts reduzierende Wirkung auf. In Figur 3 sind ferner mehrere Beleuchtungslichtstrahlen 64 in gestrichelten Linien gezeigt, um die Verteilung des Beleuchtungslichts durch die Lichtverteilungseinrichtung 40 anzudeuten.

Figur 4 zeigt eine schematische Darstellung einer weiteren Ausführungsform, die den Ausführungsformen der Figuren 2 und 3 in einigen Merkmalen ähnelt. Insbesondere ist eine Lichtverteilungseinrichtung 40 vorgesehen, die mehrere Prismen und mehrere reflektierende Flächen umfasst. Im Sinne einer übersichtlichen Darstellung sind in Figur 4 einige Merkmale, die entsprechenden Merkmalen der Ausführungsform der Figur 2 ähneln, nicht mit Bezugszeichen versehen.

Die Ausführungsform der Figur 4 unterscheidet sich von den Ausführungsformen der Figuren 2 und 3 unter anderem dadurch, dass ein Kollimator 61 vorgesehen ist, der mit den Prismen der Lichtverteilungseinrichtung 40 gefügt ist bzw. Bestandteil der Lichtverteilungseinrichtung 40 ist. Deshalb ist die Lichteintrittsfläche 47 der Lichtverteilungseinrichtung 40 gleichzeitig die Lichteintrittsfläche des Kollimators 61. Die Grenzfläche zwischen dem Kollimator 61 und den Prismen der Lichtverteilungseinrichtung 40 kann wie in Figur 4 angedeutet abschnittsweise eben oder abweichend von der Darstellung in Figur 4 kontinuierlich gekrümmt oder insgesamt eben sein.

Die in Figur 4 dargestellte Lichtverteilungseinrichtung 40 unterscheidet sich von den Ausführungsformen der Figuren 2 und 3 ferner dadurch, dass die Lichtaustrittsfläche 48 der Lichtverteilungseinrichtung 40 nicht abschnittsweise eben, sondern abschnittsweise konvex gekrümmt ist. Insbesondere umfasst die Lichtaustrittsfläche 48 mehrere jeweils an ein einziges Prisma angrenzende sphärische, elliptische, torische oder zylindrische Abschnitte. Mit der Krümmung der Lichtaustrittsfläche 48 bzw. der an die einzelnen Prismen angrenzenden Abschnitte der Lichtaustrittsfläche 48 kann die Verteilung des aus der Lichtverteilungseinrichtung 40 austretenden Beleuchtungslichts eingestellt werden. Abweichend von der Darstellung in Figur 4 kann die Lichtaustrittsfläche 48 abschnittsweise konkav sein.

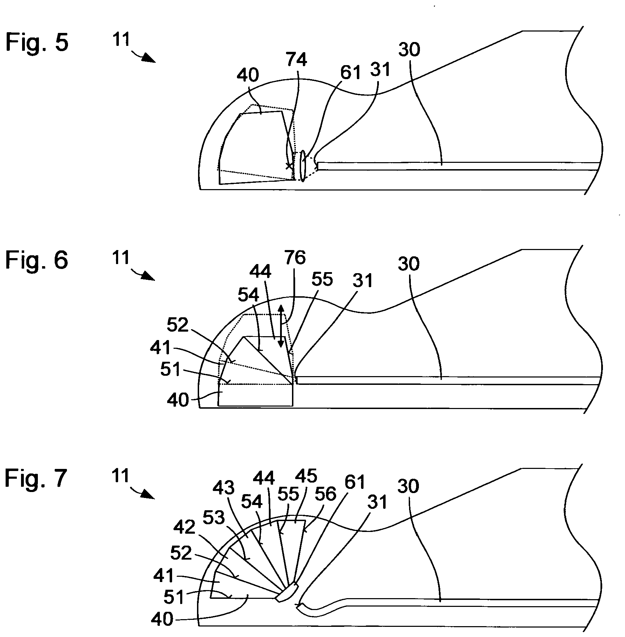

Figur 5 zeigt eine schematische Darstellung einer weiteren Ausführungsform, die in einigen Merkmalen den Ausführungsformen der Figur 2 bis 4 ähnelt, insbesondere der Ausführungsform der Figur 2. Insbesondere sind ein Kollimator 61 und eine Lichtverteilungseinrichtung 40 vorgesehen, die der Ausführungsform der Figur 2 entsprechen. Im Sinne einer übersichtlichen Darstellung sind in Figur 5 einige Merkmale, die entsprechenden Merkmalen der Ausführungsform der Figur 2 ähneln, nicht mit Bezugszeichen versehen. Aus entsprechendem Grund sind die reflektierenden Flächen der Lichtverteilungseinrichtung 40 nicht dargestellt.

Abweichend von der Ausführungsform der Figur 2 ist die Lichtverteilungseinrichtung 40 um eine Schwenkachse 74 schwenkbar. Die Schwenkachse 74 ist senkrecht zur Zeichenebene der Figur 5 und insbesondere parallel zur in Figur 1 dargestellten Schwenkachse der Blickrichtung des Endoskops. Die Kontur der Lichtverteilungseinrichtung 40 ist in Figur 5 in zwei verschiedenen Positionen dargestellt, einmal in durchgezogener Linie und einmal in unterbrochener Linie.

Durch Schwenken der Lichtverteilungseinrichtung 40 um die Schwenkachse 74 kann die Verteilung des Beleuchtungslichts verändert werden. Bei der Ausführungsform der Figur 5 ist die Schwenkachse 74 nahe an der Lichteintrittsfläche der Lichtverteilungseinrichtung 40 angeordnet. Abweichend davon kann die Schwenkachse an einem anderen Ort innerhalb oder außerhalb der Kontur der Lichtverteilungseinrichtung 40 angeordnet sein.

Figur 6 zeigt eine schematische Darstellung einer weiteren Ausführungsform, die in einigen Merkmalen der Ausführungsform der Figur 2 ähnelt. Insbesondere ist eine Lichtverteilungseinrichtung 40 vorgesehen, die der oben anhand der Figur 2 dargestellten Lichtverteilungseinrichtung entspricht. Im Sinne einer übersichtlichen Darstellung sind in Figur 2 einige Merkmale, die entsprechenden Merkmalen der Ausführungsform der Figur 2 ähneln, nicht mit Bezugszeichen versehen. Aus entsprechendem Grund sind die reflektierenden Flächen der Lichtverteilungseinrichtung 40 nur teilweise dargestellt.

Im Gegensatz zu der Ausführungsform der Figur 2 ist kein Kollimator vorgesehen. Die Ausführungsform der Figur 6 unterscheidet sich von der Ausführungsform der Figur 2 ferner dadurch, dass die Lichtverteilungseinrichtung in einer Richtung 76 verfahrbar ist. Die Richtung 76 ist insbesondere senkrecht zur oben anhand der Figur 1 dargestellten Längsachse des Endoskops und parallel zur Zeichenebene der Figur 6. Alternativ kann die Lichtverteilungseinrichtung 40 entlang eines gekrümmten Wegs verfahrbar sein.

Die Lichtverteilungseinrichtung 40 ist in Figur 6 in zwei Positionen dargestellt, einmal in durchgezogener Linie und einmal in unterbrochener Linie. Im Sinne einer übersichtlichen Darstellung sind neben der Kontur der Lichtverteilungseinrichtung 40 jeweils nur zwei reflektierende Flächen 51, 52 bzw. 54, 55 dargestellt und mit Bezugszeichen versehen.

Aufgrund des geringen Abstands zwischen der Lichtaustrittsfläche 31 des Lichtleiters 30 und der Lichteintrittsfläche der Lichtverteilungseinrichtung 40 und der im Verhältnis zur Lichteintrittsfläche der Lichtverteilungseinrichtung 40 kleinen Lichtaustrittsfläche 31 des Lichtleiters 30 wird aus der Lichtaustrittsfläche 31 des Lichtleiters 30 austretendes Beleuchtungslicht bei den beiden dargestellten Positionen der Lichtverteilungseinrichtung 40 jeweils überwiegend nur in ein Prisma der Lichtverteilungseinrichtung 40 eingekoppelt. Bei der in unterbrochener Linie dargestellten Position der Lichtverteilungseinrichtung 40 wird Beleuchtungslicht überwiegend in das Prisma 41 zwischen den reflektierenden Flächen 51, 52 eingekoppelt, bei der in durchgezogener Linie dargestellten Position der Lichtverteilungseinrichtung 40 wird Beleuchtungslicht überwiegend in das Prisma 44 zwischen den reflektierenden Flächen 54, 55 eingekoppelt.

Durch Verschieben der Lichtverteilungseinrichtung 40 entlang eines geraden oder - abweichend von der Darstellung in Figur 6 - gekrümmten Wegs kann so die Verteilung des Beleuchtungslichts beeinflusst werden. Die Verteilung des Beleuchtungslichts kann insbesondere dadurch beeinflusst werden, dass das Beleuchtungslicht in unterschiedliche Prismen eingekoppelt wird uns in der Folge an reflektierenden Flächen reflektiert wird, die unterschiedlich ausgerichtet sind.

Figur 7 zeigt eine schematische Darstellung einer weiteren Ausführungsform, die in einigen Merkmalen den oben anhand der Figuren 2 bis 4 dargestellten Ausführungsformen ähnelt. Insbesondere ist eine Lichtverteilungseinrichtung 40 vorgesehen, die mehrere Prismen 41, 42, 43, 44, 45 und mehrere reflektierende Flächen 51, 52, 53, 54, 55, 56 aufweist. Ähnlich wie bei der Ausführungsform der Figur 4 ist ein Kollimator 61 vorgesehen, der mit den Prismen 41, 42, 43, 44, 45 der Lichtverteilungseinrichtung 40 gefügt bzw. Bestandteil der Lichtverteilungseinrichtung 40 ist. Abweichend von der Ausführungsform der Figur 4 ist die Lichtverteilungseinrichtung 40 einschließlich des Kollimators 61 im Wesentlichen symmetrisch zu einer Ebene, die zur Zeichenebene der Figur 7 senkrecht ist und die Längsachse des Endoskops bei dem dargestellten Beispiel in einem Winkel von 40 Grad bis 50 Grad schneidet. Ferner weist der Lichtleiter 30 am distalen Ende eine Krümmung auf. Die Lichtaustrittsfläche 31 des Lichtleiters 30 ist so angeordnet und ausgerichtet, dass aus der Lichtaustrittsfläche 31 des Lichtleiters 30 austretendes Beleuchtungslicht auf den Kollimator 61 fällt.

Einige Merkmale der oben anhand der Figuren 2 bis 7 dargestellten Ausführungsformen sind in anderer Weise miteinander kombinierbar, beispielsweise die konvexe oder konkave Krümmung der Lichteintrittsfläche, die konvexe oder konkave Krümmung der Lichtaustrittsfläche der Lichtverteilungseinrichtung, die Ausführungsform und die Anordnung des Kollimators, die Schwenkbarkeit bzw. Verschiebbarkeit und die Krümmung des Lichtleiters nahe seiner Lichtaustrittsfläche. Ferner kann abweichend von der Darstellung in den Figuren 2 bis 5 und 7 auf einen Kollimator oder eine kollimierend wirkende konvexe Lichteintrittsfläche der Lichtverteilungseinrichtung verzichtet werden, um eine andere Verteilung des Beleuchtungslichts zu bewirken. Insbesondere kann alternativ eine Linse oder eine entsprechende Krümmung der Lichteintrittsfläche der Lichtverteilungseinrichtung vorgesehen sein, die die Divergenz des aus der Lichtaustrittsfläche des Lichtleiters austretenden Beleuchtungslichts nicht oder nicht wesentlich verringert oder sogar vergrößert.

Bei allen oben anhand der Figuren 2 bis 7 dargestellten Ausführungsformen kann anstelle einer Kupplung zum Koppeln des Endoskops mit einer externen Lichtquelle eine interne Lichtquelle am proximalen Ende des Endoskops vorgesehen sein. Ferner kann alternativ anstelle des Lichtleiters 30 eine Lichtquelle am distalen Ende 11 des Endoskops vorgesehen sein.

Abweichend von den oben anhand der Figuren 2 bis 7 dargestellten Ausführungsformen kann die Lichtverteilungseinrichtung anstelle von Prismen nicht-prismatische transparente Körper umfassen. Dadurch sind insbesondere in einer oder in zwei Richtungen gekrümmte reflektierende Flächen zwischen den transparenten Körpern realisierbar.

Ferner kann die Lichtverteilungseinrichtung abweichend von den obigen Darstellungen anhand der Figuren 2 bis 7 keine Prismen aufweisen. In diesem Fall sind die reflektierenden Flächen beispielsweise ähnlich wie bei herkömmlichen haushaltsüblichen Spiegeln an dünnen Glastafeln angeordnet, die einseitig oder beidseitig verspiegelt sein können.

### Bezugszeichen

- 10: Endoskop
- 11: distales Ende des Endoskops 10
- 12: proximales Ende des Endoskops 10
- 14: Schaft des Endoskops 10
- 15: erste Kupplung
- 16: zweite Kupplung
- 18: Längsachse des Endoskops 10
- 20: Oberfläche eines Fensterbauteils
- 21: erste, extreme Blickrichtung (0°)
- 22: zweite, extreme Blickrichtung (120°)
- 28: Schwenkachse der Blickrichtung
- 29: Winkelbereich der Blickrichtungen
- 30: Lichtleiter
- 31: Lichtaustrittfläche am distalen Ende des Lichtwellenleiters 30

- 40: Lichtverteilungseinrichtung
- 41: erstes Prisma der Lichtverteilungseinrichtung 40
- 42: zweites Prisma der Lichtverteilungseinrichtung 40
- 43: drittes Prisma der Lichtverteilungseinrichtung 40
- 44: viertes Prisma der Lichtverteilungseinrichtung 40
- 45: fünftes Prisma der Lichtverteilungseinrichtung 40
- 47: Lichteintrittsfläche der Lichtverteilungseinrichtung 40
- 48: Lichtaustrittsfläche der Lichtverteilungseinrichtung 40
- 51: erste reflektierende Fläche der Lichtverteilungseinrichtung 40
- 52: zweite reflektierende Fläche der Lichtverteilungseinrichtung 40
- 53: dritte reflektierende Fläche der Lichtverteilungseinrichtung 40
- 54: vierte reflektierende Fläche der Lichtverteilungseinrichtung 40
- 55: fünfte reflektierende Fläche der Lichtverteilungseinrichtung 40
- 56: sechste reflektierende Fläche der Lichtverteilungseinrichtung 40

- 61: Kollimator
- 62: Linsenbauteil
- 64: Beleuchtungs-Lichtstrahl

- 70: Beleuchtungsstrahlengang
- 71: Lichtausbreitungsrichtung im Beleuchtungsstrahlengang 70
- 72: Lichtausbreitungsrichtung im Beleuchtungsstrahlengang 70
- 73: Lichtausbreitungsrichtung im Beleuchtungsstrahlengang 70
- 74: Schwenkachse der Lichtverteilungseinrichtung 40
- 76: Richtung der Verfahrbarkeit der Lichtverteilungseinrichtung 40

## Patentansprüche

1. **Endoskop** (10) **mit** relativ zum Endoskop **schwenkbarer Blickrichtung** (21, 22), mit:
einem **Beleuchtungsstrahlengang** (70) eingerichtet zum Übertragen von Beleuchtungslicht;
einer Mehrzahl von **reflektierenden Flächen** (51, 52, 53, 54, 55, 56) eingerichtet zum Reflektieren des Beleuchtungslichts, wobei die reflektierenden Flächen (51, 52, 53, 54, 55, 56) bezogen auf die vorgesehene Lichtausbreitungsrichtung (71, 72, 73) von Beleuchtungslicht im Beleuchtungsstrahlengang (70) zumindest teilweise **nebeneinander** im Beleuchtungsstrahlengang (70) angeordnet sind,
**dadurch gekennzeichnet, dass** die reflektierenden Flächen (51, 52, 53, 54, 55, 56) **fächerförmig** angeordnet sind, weiterhin **gekennzeichnet durch** eine **Lichtverteilungseinrichtung** (40), die eine Mehrzahl aneinander angrenzender transparenter Körper (41, 42, 43, 44, 45) umfasst, wobei die reflektierenden Flächen (51, 52, 53, 54, 55, 56) an Grenzflächen zwischen jeweils zwei der aneinander angrenzenden transparenten Körpern (41, 42, 43, 44, 45) vorliegen.

2. Endoskop (10) nach einem der vorangehenden Ansprüche, bei dem **Flächennormalen** ebener reflektierender Flächen (51, 52, 53, 54, 55, 56) der Mehrzahl von reflektierenden Flächen (51, 52, 53, 54, 55, 56) **in einer Ebene** senkrecht zu einer Schwenkachse (28) der Blickrichtung (21, 22) liegen.

3. Endoskop (10) nach einem der vorangehenden Ansprüche, bei dem lokale **Flächennormalen** reflektierender Flächen (51, 52, 53, 54, 55, 56) in mittleren Bereichen der reflektierenden Flächen (51, 52, 53, 54, 55, 56) in einer Ebene senkrecht zu einer Schwenkachse (28) der Blickrichtung (21, 22) liegen.

4. Endoskop (10) nach einem der vorangehenden Ansprüche, bei dem eine reflektierende Fläche (52, 53, 54, 55) der Mehrzahl von reflektierenden Flächen (51, 52, 53, 54, 55, 56) einen **Reflexionsgrad** von höchstens 0,75 aufweist.

5. Endoskop (10) nach dem vorangehenden Anspruch, bei dem die transparenten Körper **Prismen** (41, 42, 43, 44, 45) aus einem transparenten Material sind.

6. Endoskop (10) nach einem der Ansprüche 1-5, bei dem an einzelne transparente Körper (41, 42, 43, 44, 45) der Lichtverteilungseinrichtung (40) angrenzende Abschnitte einer **Lichtaustrittsfläche** (48) der Lichtverteilungseinrichtung (40) eben sind.

7. Endoskop (10) nach einem der Ansprüche 1-5, bei dem an einzelne transparente Körper (41, 42, 43, 44, 45) der Lichtverteilungseinrichtung (40) angrenzende Abschnitte einer **Lichtaustrittsfläche** (48) der Lichtverteilungseinrichtung (40) **gekrümmt** sind.

8. Endoskop (10) nach einem der Ansprüche 1-7, bei dem an einzelne transparente Körper (41, 42, 43, 44, 45) der Lichtverteilungseinrichtung (40) angrenzende Abschnitte einer **Lichteintrittsfläche** (47) der Lichtverteilungseinrichtung (40) eben sind.

9. Endoskop (10) nach einem der vorangehenden Ansprüche, bei dem die reflektierenden Flächen (51, 52, 53, 54, 55, 56) zumindest entweder um eine Achse (74) **schwenkbar** oder entlang eines Wegs (76) **verschiebbar** sind.

10. Endoskop (10) nach dem vorangehenden Anspruch, bei dem die Beleuchtung der reflektierenden Flächen (51, 52, 53, 54, 55, 56) abhängig von der durch Schwenken oder Schieben der reflektierenden Flächen (51, 52, 53, 54, 55, 56) eingestellten Position der reflektierenden Fläche (51, 52, 53, 54, 55, 56) ist.

## Claims

1. Endoscope (10) having a viewing direction (21, 22) which can be pivoted relative to the endoscope, comprising:
an illumination beam path (70) configured for transmitting illumination light;
a multiplicity of reflective surfaces (51, 52, 53, 54, 55, 56) configured for reflecting the illumination light, the reflective surfaces (51, 52, 53, 54, 55, 56) being arranged at least partially next to one another in the illumination beam path (70) relative to the intended light propagation direction (71, 72, 73) of illumination light in the illumination beam path (70), **characterized in that** reflective surfaces (51, 52, 53, 54, 55, 56) are arranged in the shape of a fan, furthermore **characterized by** a light distribution device (40) which comprises a multiplicity of adjacent transparent bodies (41, 42, 43, 44, 45), the reflective surfaces (51, 52, 53, 54, 55, 56) being present at interfaces respectively between two of the adjacent transparent bodies (41, 42, 43, 44, 45).

2. Endoscope (10) according to one of the preceding claims, wherein surface normals of planar reflective surfaces (51, 52, 53, 54, 55, 56) of the multiplicity of reflective surfaces (51, 52, 53, 54, 55, 56) lie in a plane perpendicular to a pivot axis (28) of the viewing direction (21, 22).

3. Endoscope (10) according to one of the preceding claims, wherein local surface normals of reflective surfaces (51, 52, 53, 54, 55, 56) in the central regions of the reflective surfaces (51, 52, 53, 54, 55, 56) lie in a plane perpendicular to a pivot axis (28) of the viewing direction (21, 22).

4. Endoscope (10) according to one of the preceding claims, wherein a reflective surface (52, 53, 54, 55) of the multiplicity of reflective surfaces (51, 52, 53, 54, 55, 56) has a reflectance of at most 0.75.

5. Endoscope (10) according to the preceding claim, wherein the transparent bodies are prisms (41, 42, 43, 44, 45) made of a transparent material.

6. Endoscope (10) according to one of Claims 1 to 5, wherein sections of a light exit surface (48) of the light distribution device (40), which adjoin individual transparent bodies (41, 42, 43, 44, 45) of the light distribution device (40), are planar.

7. Endoscope (10) according to one of Claims 1 to 5, wherein sections of a light exit surface (48) of the light distribution device (40), which adjoin individual transparent bodies (41, 42, 43, 44, 45) of the light distribution device (40), are curved.

8. Endoscope (10) according to one of Claims 1 to 7, wherein sections of a light entry surface (47) of the light distribution device (40), which adjoin individual transparent bodies (41, 42, 43, 44, 45) of the light distribution device (40), are planar.

9. Endoscope (10) according to one of the preceding claims, wherein the reflective surfaces (51, 52, 53, 54, 55, 56) are at least either pivotable about an axis (74) or displaceable along a path (76).

10. Endoscope (10) according to the preceding claim, wherein the illumination of the reflective surfaces (51, 52, 53, 54, 55, 56) is dependent on the position of the reflective surfaces (51, 52, 53, 54, 55, 56) adjusted by pivoting or displacing the reflective surfaces (51, 52, 53, 54, 55, 56).

## Revendications

1. Endoscope (10) présentant une direction de visée (21, 22) basculante par rapport à l'endoscope, comportant :
un trajet de faisceau d'éclairage (70) conçu pour la transmission de la lumière d'éclairage ;
une pluralité de surfaces réfléchissantes (51, 52, 53, 54, 55, 56) conçues pour réfléchir la lumière d'éclairage,
dans lequel les surfaces réfléchissantes (51, 52, 53, 54, 55, 56) sont disposées au moins en partie les unes à côté des autres sur le trajet du faisceau d'éclairage (70) par rapport à la direction de propagation de lumière (71, 72, 73) prévue de la lumière d'éclairage sur le trajet du faisceau d'éclairage (70),
**caractérisé en ce que** les surfaces réfléchissantes (51, 52, 53, 54, 55, 56) sont disposées en éventail, et **caractérisé en outre par** un dispositif de répartition de lumière (40) comprenant une pluralité de corps transparents adjacents les uns aux autres (41, 42, 43, 44, 45), dans lequel les surfaces réfléchissantes (51, 52, 53, 54, 55, 56) sont respectivement présentes aux interfaces entre deux des corps transparents adjacents les uns aux autres (41, 42, 43, 44, 45).

2. Endoscope (10) selon l'une des revendications précédentes, dans lequel des normales à des surfaces réfléchissantes planes (51, 52, 53, 54, 55, 56) de la pluralité de surfaces réfléchissantes (51, 52, 53, 54, 55, 56) se situent dans un plan perpendiculaire à un axe de pivotement (28) de la direction de visée (21, 22) .

3. Endoscope (10) selon l'une des revendications précédentes, dans lequel des normales locales à des surfaces réfléchissantes (51, 52, 53, 54, 55, 56) dans des régions centrales des surfaces réfléchissantes (51, 52, 53, 54, 55, 56) se situent dans un plan perpendiculaire à un axe de pivotement (28) de la direction de visée (21, 22).

4. Endoscope (10) selon l'une des revendications précédentes, dans lequel une surface réfléchissante (52, 53, 54, 55) de la pluralité de surfaces réfléchissantes (51, 52, 53, 54, 55, 56) présente un coefficient de réflexion d'au plus 0,75.

5. Endoscope (10) selon la revendication précédente, dans lequel les corps transparents sont des prismes (41, 42, 43, 44, 45) constitués d'un matériau transparent.

6. Endoscope (10) selon l'une des revendications 1-5, dans lequel des parties d'une surface de sortie de lumière (48) du dispositif de répartition de lumière (40) qui sont adjacentes à des corps transparents (41, 42, 43, 44, 45) individuels du dispositif de répartition de lumière (40) sont planes.

7. Endoscope (10) selon l'une des revendications 1 à 5, dans lequel des parties d'une surface de sortie de lumière (48) du dispositif de répartition de lumière (40) qui sont adjacentes à des corps transparents (41, 42, 43, 44, 45) individuels du dispositif de répartition de lumière (40) sont incurvées.

8. Endoscope (10) selon l'une des revendications 1-7, dans lequel des parties d'une surface d'entrée de lumière (47) du dispositif de répartition de lumière (40) qui sont adjacentes à des corps transparents (41, 42, 43, 44, 45) individuels du dispositif de répartition de lumière (40) sont planes.

9. Endoscope (10) selon l'une des revendications précédentes, dans lequel les surfaces réfléchissantes (51, 52, 53, 54, 55, 56) sont au moins soit pivotantes autour d'un axe (74) soit déplaçables le long d'un trajet (76).

10. Endoscope (10) selon la revendication précédente, dans lequel l'éclairage des surfaces réfléchissantes (51, 52, 53, 54, 55, 56) dépend de la position de la surface réfléchissante (51, 52, 53, 54, 55, 56) qui est réglée par pivotement ou déplacement des surfaces réfléchissantes (51, 52, 53, 54, 55, 56).
